# EUROPEAN PATENT APPLICATION

(11) **EP 4 756 832 A1**
(43) Date of publication of application: **10.06.2026**
(21) Application number: 25221293.1
(22) Date of filing: 05.12.2025
(51) Int. Cl.: G16H 40/63

(54) **PROGRESSIVE ADVANCEMENT OF AUTOMATED LEVEL BASED ON LEARNED COMPLIMENTARY ASSISTANCE**

(30) Priority: 06.12.2024 US 202418971590
(71) Applicant: Cilag GmbH International, 6300 Zug (CH)
(72) Inventor: SHELTON, IV, Frederick E, Ohio, 45242 (US); HARRIS, Jason L., Ohio, 45242 (US)
(74) Representative: Carpmaels & Ransford LLP

(57) **Abstract**

A system may adaptively recognize the abilities of surgical elements to adjust an automation strategy during a procedure. The system may be configured to obtain operational data for a first surgical element and a second surgical element. The system may determine an automation assistance parameter. The system may detect an adaptive recognition event. The adaptive recognition event may be at least one of a relationship between the first surgical element and the second surgical element, or a determination that the physiological parameter of the patient satisfies a life-threatening threshold. The system may select a second automation assistance parameter for the first surgical element. The system may transmit an indication of the second automation assistance parameter to the first surgical element. The system may cause the first surgical element to perform the second set of automated tasks based on the second automation assistance parameter.

## Description

### CROSS-REFERENCE TO RELATED APPLICATIONS

This application is related to the following, filed contemporaneously, the contents of each of which are incorporated by reference herein:
- Attorney Docket No. END9638USNP2, entitled ADJUSTING AUTOMATED COOPERATIVE OPERATIONS BASED ON SITUATIONALLY DERIVED CONSTRAINTS,
- Attorney Docket No. END9638USNP3, entitled ASSISTANCE ADVANCEMENT MULTI-SYSTEM INTERACTION,
- Attorney Docket No. END9638USNP4, entitled MONITORING AND IDENTIFYING SURGEON CONTROL AND SUGGESTING A TASK THAT MAY BE DONE AUTONOMOUSLY,
- Attorney Docket No. END9638USNP5, entitled CONTROL OF INFORMATION FLOW, PRIORITIZATION AND MANIFESTATION OF DATA ASSOCIATED WITH AN ACTIVE HCP INTERACTION SPACE,
- Attorney Docket No. END9638USNP6, entitled ADAPTIVE RETRACTION FORCE CONTROL,
- Attorney Docket No. END9638USNP7, entitled ADJUSTMENT OR DISPLAY OF OPTIONS OF POSITIONAL OR ORIENTATION IMPLICATIONS ON SURGICAL TOOL USAGE, and
- Attorney Docket No. END9638USNP8, entitled ADJUSTMENT OF PHYSIOLOGIC FUNCTION SUPPLEMENTATION CONTROL.

The contents of each of the following are incorporated by reference herein:
- U.S. Patent Application No. 18/810,323 entitled METHOD FOR MULTI-SYSTEM INTERACTION, filed on August 20, 2024;
- U.S. Patent Application No. 18/960,006 entitled METHOD FOR SMART SURGICAL SYSTEMS filed on November 26, 2024; and
- U.S. Patent Application No. 18/954,186 entitled METHOD FOR MULTI-SYSTEM INTERACTION, filed on November 20, 2024.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

Automated levels may be progressively advanced based on learned complementary assistance to reduce manual tasks performed by an HCP, improve efficiency and patient safety, and further optimize a procedure. An automation strategy selector system (referred to herein as a "system") may obtain operational data from one or more smart devices, an HCP, and/or a server. The operational data may include an indication of a physiological parameter of a patient during a procedure and/or a plurality of automated tasks associated with the procedure. The system may determine one or more tasks that may be automated during a procedure based on operational data. The system may detect an adaptive recognition event. An adaptive recognition event may include a relationship between smart devices and/or a determination that the physiological parameter of the patient satisfies a threshold (e.g., a life-threatening threshold).

Based on the adaptive recognition event, the system may select a second automation assistance parameter for a smart device. The automation assistance parameter may indicate a task from a set of automated tasks that may be performed by the smart device during a procedure. For example, if the system detects that a physiological parameter satisfies a life-threatening threshold, the system may select an automation assistance parameter that increases the level of automation and/or to fully automates tasks associated with a procedure. In examples, if the system detects a relationship between the surgical elements, the system may select an automation assistance parameter that optimizes the procedure based on the determined relationship (e.g., automates selected tasks to reduce distractions, focus workload to critical tasks, and/or eliminate the potential for miscommunication during a procedure). The system may transmit an indication of the automation assistance parameter to a smart device (e.g., and/or an instruction), to cause the smart device to perform tasks associated with the automation assistance parameter.

Systems, methods, and/or instrumentalities disclosed herein may be related to progressive advancement of automated levels based on learned complementary assistance. A system may be configured to adaptively recognize an automation strategy during a procedure. The system may include a processor. The system may obtain operational data for a first surgical element and/or a second surgical element. The operational data may include an indication of a physiological parameter of a patient during the procedure and/or a plurality of automated tasks associated with the procedure. The system may determine an automation assistance parameter for the first surgical element. The automation assistance parameter may be associated with a set of automated tasks from the plurality of automated tasks that are performed by the first surgical element during the procedure. The system may detect an adaptive recognition event. The adaptive recognition event may be at least one of: a relationship between the first surgical element and the second surgical element, or a determination that the physiological parameter of the patient satisfies a life-threatening threshold. The system may, based on the adaptive recognition event, select a second automation assistance parameter for the first surgical element. The second automation assistance parameter may indicate a second set of automated tasks that are performed by the first surgical element during the procedure. The system may transmit an indication of the second automation assistance parameter to the first surgical element. The system may cause the first surgical element to perform the second set of automated tasks based on the second automation assistance parameter.

One or more features may be included. For example, the first surgical element may be a ventilator. The second surgical element may be a heating device. The physiological parameter of a patient may be a CO2 percentage from the ventilator or a core body temperature from the heating device. The procedure of the patient may include cardiac atrial fibrillation (AFIB) nerve ablation. The adaptive recognition event may include a determination that a CO2 to O2 or a core body temperature of the patient satisfies the life-threatening threshold. The second automation assistance parameter may include an instruction to decrease an O2 supplementation level of the ventilator or an instruction to increase a tidal volume of the ventilator.

For example, the physiological parameter of the patient may satisfy a life-threatening threshold if the first surgical element or the second surgical element determines that a heart rate exceeds an operational window of 40-120 beats per minute, an oxygen saturation of the patient decreases below 90%, or a core body temperature of the patient is less than 89 degrees Fahrenheit. The adaptive recognition event may include the determination that the physiological parameter of the patient satisfies the life-threatening threshold. The selected second automation assistance parameter may indicate that the second set of automated tasks includes the plurality of automated tasks. The adaptive recognition event may include the relationship between the first surgical element and the second surgical element. The selected second automation assistance parameter may include an instruction to perform the second set of automated tasks in response to a user input.

For example, the system may send an automation strategy message to a user. The automation strategy message may include a request to perform the second set of automated tasks. The system may receive a user input including an instruction to perform at least one automated task of the second set of automated tasks. The system may receive position data associated with a movement of a user during the procedure and/or control data associated with the first surgical element and/or the second surgical element. The system may determine the relationship between the first surgical element and the second surgical element based on the position data, the operational data, and/or the physiological parameter of the patient. The system may, based on the adaptive recognition event, select an automation assistance parameter for the second surgical element. The automation assistance parameter for the second surgical element may indicate a set of automated tasks to be performed by the second surgical element during the procedure. The system may transmit, to the second surgical element, an indication of the automation assistance parameter for the second surgical element. The system may cause the second surgical element to perform the set of automated tasks based on the automation assistance parameter for the second surgical element.

A method for adaptively recognizing an automation strategy during a procedure may be described. The method may include obtaining operational data for a first surgical element and/or a second surgical element. The operational data may include an indication of a physiological parameter of a patient during the procedure and/or a plurality of automated tasks associated with the procedure. The method may include determining an automation assistance parameter for the first surgical element. The automation assistance parameter may be associated with a set of automated tasks from the plurality of automated tasks that are performed by the first surgical element during the procedure. The method may include detecting an adaptive recognition event. The adaptive recognition event may be at least one of: a relationship between the first surgical element and the second surgical element or a determination that the physiological parameter of the patient satisfies a life-threatening threshold. The method may include, based on the adaptive recognition event, selecting a second automation assistance parameter for the first surgical element. The second automation assistance parameter may indicate a second set of automated tasks that are performed by the first surgical element during the procedure. The method may include transmitting an indication of the second automation assistance parameter to the first surgical element. The method may include causing the first surgical element to perform the second set of automated tasks based on the second automation assistance parameter.

The method may include one or more features. For example, the first surgical element may be a ventilator. The second surgical element may be a heating device. The physiological parameter of a patient may be a CO2 percentage from the ventilator or a core body temperature from the heating device. The procedure of the patient may include a cardiac AFIB nerve ablation. The adaptive recognition event may include a determination that a CO2 to O2 or a core body temperature of the patient satisfies the life-threatening threshold. The second automation assistance parameter may include an instruction to decrease an O2 supplementation level of the ventilator or an instruction to increase a tidal volume of the ventilator.

For example, the adaptive recognition event may include the determination that the physiological parameter of the patient satisfies the life-threatening threshold. The selected second automation assistance parameter may indicate that the second set of automated tasks includes the plurality of automated tasks. The adaptive recognition event may include the relationship between the first surgical element and the second surgical element. The selected second automation assistance parameter may include an instruction to perform the second set of automated tasks in response to a user input. The method may include sending an automation strategy message to a user. The automation strategy message may include a request to perform the second set of automated tasks. The method may include receiving a user input comprising an instruction to perform at least one automated task of the second set of automated tasks. The method may include receiving position data associated with a movement of a user during the procedure and/or control data associated with the first surgical element and/or the second surgical element. The method may include determining the relationship between the first surgical element and the second surgical element based on the position data, the operational data, and/or the physiological parameter of the patient.

For example, the method may include based on the adaptive recognition event, selecting an automation assistance parameter for the second surgical element. The automation assistance parameter for the second surgical element may indicate a set of automated tasks to be performed by the second surgical element during the procedure. The method may include transmitting, to the second surgical element, an indication of the automation assistance parameter for the second surgical element. The method may include causing the second surgical element to perform the set of automated tasks based on the automation assistance parameter for the second surgical element.

A system to adaptively recognize an automation strategy may obtain operational data for a first surgical element and/or a second surgical element. The operational data may include an indication of a physiological parameter of a patient. The system may detect an adaptive recognition event based on the operational data. The system may, based on the adaptive recognition event, determine an automation assistance parameter for the first surgical element. The automation assistance parameter may indicate a set of automated tasks that are performed by the first surgical element. The system may transmit an indication of the automation assistance parameter to the first surgical element. The system may cause the first surgical element to perform the second set of automated tasks based on the automation assistance parameter.

The system may include one or more features. For example, the adaptive recognition event may include a determination that a physiological parameter of the patient satisfies a life-threatening threshold or a determined relationship between the first surgical element and the second surgical element. The system may send an automation strategy message to a user. The automation strategy message may include a request to perform an automated tasks associated with the automation assistance parameter. The system may receive a user input comprising an instruction to perform the automated task. The system may, based on the adaptive recognition event, select a second automation assistance parameter. The second automation assistance parameter may indicate a second automated task. The system may transmit, to the second surgical element, an indication of the second automation assistance parameter. The system may cause the second surgical element to perform the second automated task.

### BRIEF DESCRIPTION OF THE DRAWINGS

FIG. 1 is a block diagram of a computer-implemented surgical system.
FIG. 2 shows an example surgical system in a surgical operating room.
FIG. 3 illustrates an example surgical hub paired with various systems.
FIG. 4 shows an example situationally aware surgical system.
FIG. 5 shows an example surgical instrument.
FIG. 6 is an example operational environment in which an automation strategy selector system may determine whether to automate one or more tasks of a procedure.
FIG. 7A is a flow diagram illustrating example operations performed by the components of the operating environment of FIG. 6, to determine whether to automate one or more tasks.
FIG. 7B is a flow diagram illustrating example operations performed by the components of the operating environment of FIG. 6, to train a machine learning (ML) model.
FIG. 8 is a flow diagram depicting an example adaptive recognition detector routine.
FIG. 9 is an example implementation for determining an automation strategy.

### DETAILED DESCRIPTION

Operating rooms are becoming more sophisticated with the introduction of smart devices (e.g., interchangeably referred to herein as "surgical elements"). Smart devices may be used and/or adjusted by a health care personnel (HCP) during a procedure. Smart devices may include one or more advanced capabilities to significantly enhance the precision, safety, and efficiency of a procedure (e.g., a surgical procedure, diagnostic procedure, therapeutic procedure, preventative procedure and/or the like), while reducing the risk of complications to patients. Examples of smart devices may include robotic surgical systems, navigation systems, smart imaging systems, endoscopic and/or laparoscopic systems, harmonic scalpels, anesthesia machines, patient monitoring systems (pulse oximeters, blood pressure monitors, EKG monitors, EEG monitors, and/or the like), energy devices (e.g., electrosurgical units, laser surgery systems, and/or the like), infusion pumps, and/or the like.

Several smart devices may be connected (e.g., via a network) to generate and/or obtain operational data during a procedure. Examples of operational data may include real-time data and/or historical data associated with environmental data (e.g., a temperature, humidity, airflow rates, pressure differential, and/or air filtration associated with the operating room), the number and position of HCPs during a procedure, a procedure plan (e.g., patient data, HCP data, tasks, supplies, smart devices, staff workflow and/or the like associated with a procedure), functions of associated smart devices (e.g., an automation assistance parameter indicating task(s) associated with a procedure plan that may be automated by a smart device), and/or patient data (e.g., physiological parameters, life-threatening thresholds associated with the patient, patient health data, and/or the like).

Although smart devices may communicate via a network and generate operational data, smart devices may not be optimized to reduce an HCP's workload during a procedure. HCPs may manually adjust one or more smart devices to, for example, maintain a patient's vitals (e.g., physiological parameters) within safe operating levels (e.g., an HCP may regulate a patient's core body temperature, regulate an SpO2 level, regulate a heart rate, and/or the like by adjusting a setpoint on a ventilator, infusion pumps, heating blankets, and/or the like).

Each time an HCP performs a manual task, the HCP's focus may be diverted from executing a critical portion of a procedure, a natural-workflow may be interrupted, and/or miscommunication may result. For example, if an HCP is constantly adjusting the position of an ablation catheter, the HCP may not adequately focus on analyzing a tissue's response to applied energy.

During a high-risk procedure such as open heart surgery, repetitive manual tasks may disrupt an HCP's natural workflow by delaying the HCP's decision-making-process, which may ultimately affect patient safety by increasing the risk of complications (e.g., a failure to rapidly address bleeding). As another example, directing support staff (e.g., nurses and assistant surgeons) to manage aspects of a procedure (e.g., suction, tool positioning, and/or the like) can result in a miscommunication, potentially delaying a team's response and/or causing further interruptions during a procedure.

To reduce manual tasks performed by an HCP, improve efficiency and patient safety, and further optimize a procedure, an automation strategy selector system (referred to herein as a "system") may obtain operational data from one or more smart devices, an HCP, and/or a server. The operational data may include an indication of a physiological parameter of a patient during a procedure and/or a plurality of automated tasks associated with the procedure. The system may determine one or more tasks that may be automated during a procedure based on operational data. The system may determine an automation assistance parameter (e.g., as part of an automation assistance strategy) for one or more smart devices. The automation assistance parameter may be associated with a set of automated tasks from a plurality of automated tasks that may be performed by the smart device during a procedure. The system may detect an adaptive recognition event. An adaptive recognition event may include a relationship between smart devices and/or a determination that the physiological parameter of the patient satisfies a threshold (e.g., a life-threatening threshold). An adaptive recognition event may comprise a determination of a relationship between a first surgical element and a second surgical element. For instance, an adaptive recognition event may comprise a determination that the relationship exists, and/or a determination that the relationship has been established.

Based on the adaptive recognition event, the system may select a second automation assistance parameter for a smart device. For example, if the system detects that a physiological parameter satisfies a life-threatening threshold, the system may select a second automation assistance parameter that includes the plurality of automated tasks (e.g., to increase the level of automation and/or to fully automate the remaining tasks associated with a procedure). In examples, if the system detects a relationship between the surgical elements, the system may select an automation assistance parameter that optimizes the procedure based on the determined relationship (e.g., to reduce distractions, focus workload to critical tasks, and/or eliminate the potential for miscommunication during a procedure). The system may transmit an indication of the second automation assistance parameter to a smart device (e.g., and/or an instruction), to cause the smart device to perform tasks associated with the second automation assistance parameter.

A more detailed understanding may be had from the following description, given by way of example in conjunction with the accompanying drawings.

### Example Aspects of a Surgical System

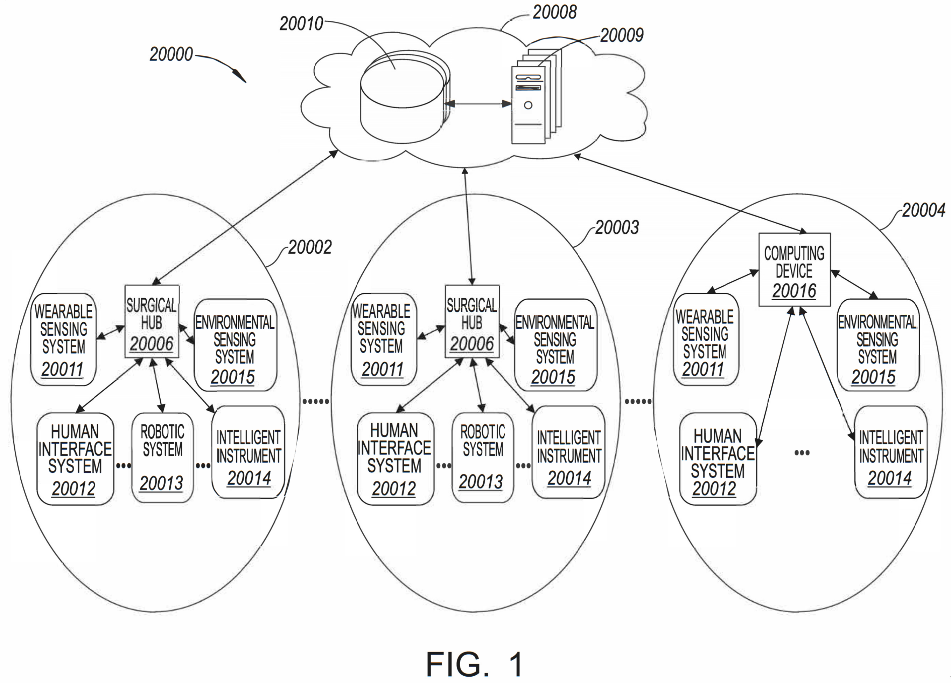

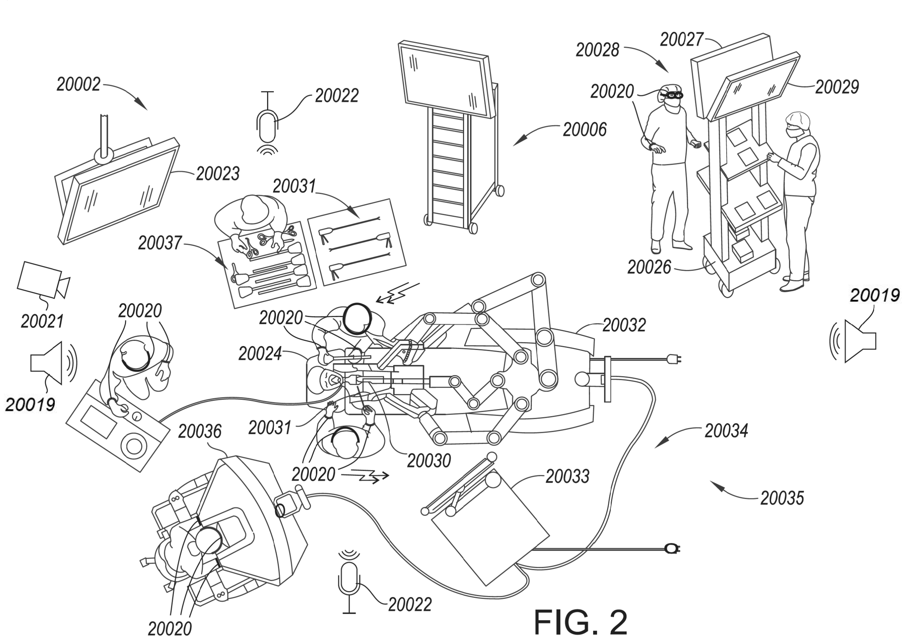

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngoneproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

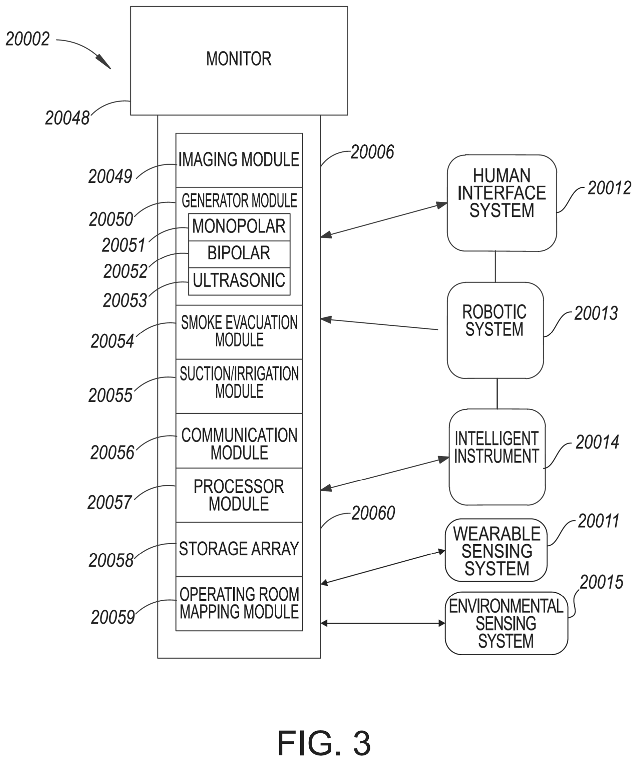

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost when addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

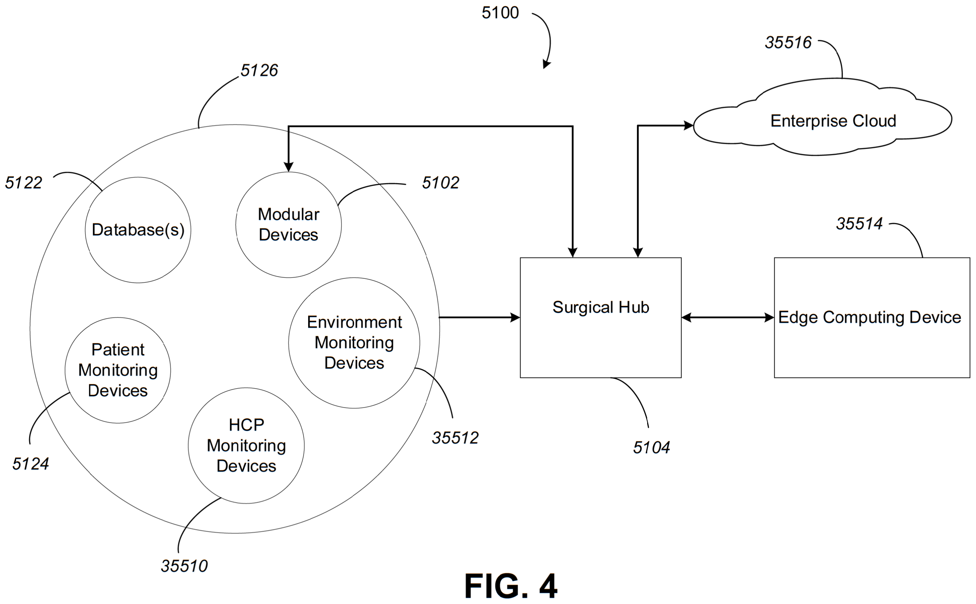

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedureby-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

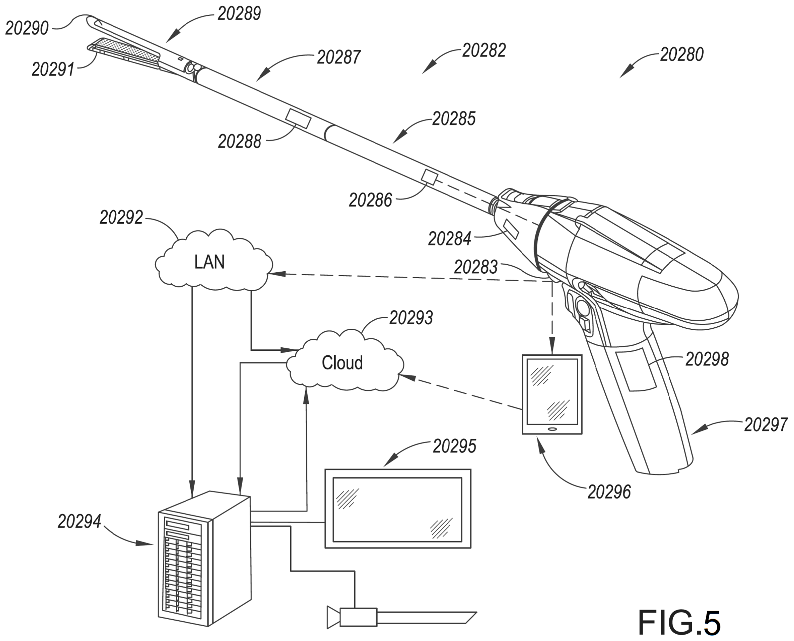

FIG. 5 illustrates an example surgical system 20280 that may include a surgical instrument 20282. The surgical instrument 20282 can be in communication with a console 20294 and/or a portable device 20296 through a local area network 20292 and/or a cloud network 20293 via a wired and/or wireless connection. The console 20294 and the portable device 20296 may be any suitable computing device. Surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreens, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

### Example Operational Environment for Determining an Automation Strategy

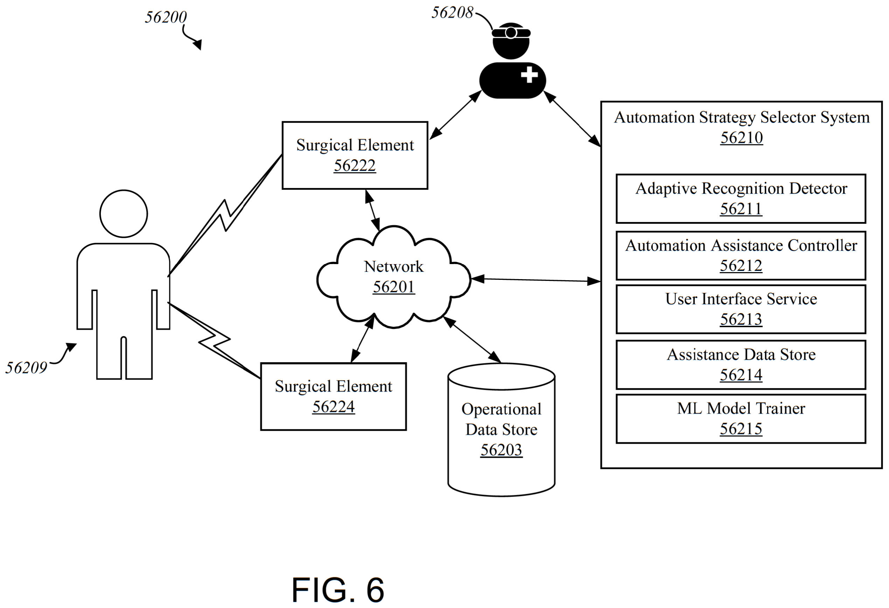

FIG. 6 is an example operational environment 56200 in which an automation strategy selector system 56210 (hereinafter referred to as a "system 56210") may, among other things, receive data from surgical element 56222, 56224, operational data store 56203, and/or HCP 56208, and determine an automation strategy including one or more automated tasks (e.g., an action, function, step, method, activity, objective, and/or the like) associated with a procedure that may be executed by a surgical element 56222, 56224.

The operation environment 56200 may include an HCP 56208, a patient 56209, an operational data store 56203, surgical element 56222, 56224, a system 56210, and network 56201. A system 56210 may receive an indication of a procedure from an HCP 56208 (e.g., via a graphical user interface (GUI)), and in response, transmit (e.g., via network 56201) a first request to surgical element 56222, 56224, to obtain a physiological parameter of a patient 56209 and a second request to an operational data store 56203 to transmit operational data associated with the procedure. The system 56210 may receive operational data (e.g., real-time or historical operational data) from surgical element 56222, 56224 and/or operational data store 56203. The system 56210 may determine, among other things, one or more tasks that may be automated by the surgical element 56222 and/or 56224 based on operational data.

An operational environment 56200 may include a system 56210. The system 56210 may include an adaptive recognition detector 56211, an automation assistance controller 56212, a user interface service 56213, assistance data store 56214, and/or ML model trainer 56215. Although the system 56210 is depicted as separate from one or more components of the operational environment 56200, the system 56210 and/or one or more components of the system 56210 may reside on a device, a server, and/or the like, such as for example, a surgical element 56222, 56224, or a surgical hub.

A system may include an adaptive recognition detector 56211 (hereinafter referred to as "detector 56211"). Detector 56211 may be for example, a processor, a controller, a field programmable gate array (FPGA), an application specific integrated circuit (ASIC), an and/or the like, configured to perform one or more actions as described herein. The detector 56211 may receive data (e.g., via network 56201) from one or more components of the operational environment 56200. For example, the detector 56211 may receive operational data from surgical elements 56222, 56224, operational data store 56203, and/or from an HCP 56208 (e.g., via user interface service 56213). A detector 56211 may transmit data to one or more components. In examples, a detector 56211 may transmit a request for information to a surgical element 56222, 56224, a request for operational data to an operational data store 56203, a request for data to an HCP 56208 (e.g., via user interface service 56213), and/or the like.

Operational data may be generated by one or more components of an operational environment 56200. Operational data may include past and/or present information associated with an operational environment 56200. Operational data may be generated based on one or more procedures (e.g., past and/or present procedures). Operational data may include: a physiological parameter of a patient (e.g., a patient's heart rate, blood pressure, SpO2, core body temperature, and/or the like); environmental data (e.g., a number of HCPs in an operating room, historical records of an HCP 56208 performing a procedure, position data associated with the movement an HCP 56208 during a procedure, a temperature, a humidity airflow rates, data associated with a hospital such as a quantity of procedures performed and/or an outcome of procedure(s) or function(s) associated with surgical elements 56222, 56224 available during a procedure, and/or the like); data associated with surgical elements 56222, 56224 (e.g., a setpoint, output characteristic, measured variable, communication data, and/or the like), user data (e.g., data generated based on a user input by an HCP 56208 via user interface service 56213), and/or the like.

As an illustrative example, operational data associated with a surgical element 56222, 56224 may include control data such as the speed of a cutting tool, a selected control loop, a voltage and/or current of an electrosurgical tool, a flow rate for an infusion pump, the RPM of a fan, a heating blanket setpoint, and/or the like. In examples, operational data may include system-generated data based on a physiological parameter of a patient, and/or other data. As an illustrative example, operational data may include a threshold (e.g., a life-threatening threshold) associated with a physiological parameter of a patient (e.g., as determined by the detector 56211). Examples of a threshold may include an operational window for a heart rate (e.g., of 40-120 beats per minute), an oxygen saturation threshold (e.g., an oxygen saturation of 92%, 90%, 88% and/or the like), or a core body temperature threshold (e.g., a core body temperature of 90, 89, 88, 87 degrees Fahrenheit) and/or the like.

A detector 56211 may determine (e.g., detect) that an adaptive recognition event (interchangeably referred to herein as an "event") has occurred. A detector 56211 may determine an event during a procedure. An event may occur based on a determination of a relationship between surgical elements 56222, 562224, a patient 56209, HCP 56208 and/or another component of the operational environment 56200. An event may occur if a physiological parameter of a patient has satisfied a threshold. Detector 56211 may transmit an indication that an event has occurred (e.g., including an indication of a relationship and/or an indication that a physiological parameter has satisfied a threshold) to one or more components of the operational environment 56200 (e.g., automation assistance controller 56212, user interface service 56213, assistance data store 56214, ML model trainer 56215, operational data store 56203, and/or surgical element 56222, 56224). In examples, detector 56211 may transmit an indication that an event has occurred to an automation assistance controller 56212 (e.g., the indication may be used by the automation assistance controller 56212 to determine whether to automate one or more tasks associated with a procedure). In examples, detector 56211 may transmit an indication that an event has occurred to a user interface service 56213, such that the user interface service 56213 may generate an alert of the event to an HCP 56208. In examples, detector 56211 may transmit an indication that an event has occurred (e.g., along with operational data) to ML model trainer 56215, to be used as training data for training an ML model to detect one or more events.

An adaptive recognition event may include a determination of a relationship (e.g., as determined by the detector 56211). Detector 56211 may determine a relationship between a first surgical element 56222, a second surgical element 56224, a patient 56209, and/or an HCP 56208. Detector 56211 may determine a relationship based on operational data (e.g., data generated during a procedure and/or based on historical operational data). A detector 56211 may determine a relationship, for example, in response to a user input (e.g., a user input from an HCP 56208 via a user interface service 56213). Detector 56211 may generate a look-up-table including one or more determined relationships and/or determine a relationship based on analysis of a look-up-table (e.g., by referencing the look-up table based on a selected procedure by an HCP 56208).

As an illustrative example, a detector 56211 may determine a relationship (e.g., based on an analysis of operational data) between a core body temperature (e.g., a setpoint measured by a surgical element 56222), a ventilator (e.g., surgical element 56224), and/or a heating blanket (e.g., one or more additional surgical elements not illustrated as part of operational environment 56200). The detector 56211 may determine a relationship, for example, based on data indicating that if a ventilator increases the tidal volume of air to a patient 56209, a heating blanket's power output may be increased to maintain the patient's core body temperature.

An adaptive recognition event may include a determination that a physiological parameter satisfies a threshold (e.g., as determined by detector 56211). The detector 56211 may receive data associated with a physiological parameter of a patient from surgical element 56222, 56224, and compare the data to a threshold. A threshold may be determined by the system 56210, determined by an HCP 56208 (e.g., received via a user input), determined by an ML model, and/or based on operational data associated with the patient 56209. In examples, a detector 56211 may determine that an event has occurred based on a condition that one, two, three, four and/or more physiological parameters satisfy a threshold. A detector 56211 may determine one or more aspects of a threshold based on operational data (e.g., data associated with a procedure, aspects of the surgical element(s) 56222, 56224 available during the procedure, an HCP 56208, and/or a patient 56209). Aspects of a threshold may include a threshold type (e.g., heart rate, SpO2, and/or the like), a threshold value (e.g., 90 degrees Fahrenheit for a core body temperature, a range for a heart rate, and/or the like), and/or the quantity of threshold(s) (1, 2, 3, 4, etc.) may vary for a procedure.

A system 56210 may include an automation assistance controller 56212 (referred to herein as "controller 56212"). Controller 56212 may be for example, a processor, a controller, an FPGA, an ASIC, and/or the like, configured to perform one or more actions as described herein. The controller 56212 may receive data (e.g., via network 56201) from one or more components of the operational environment 56200. For example, the controller 56212 may receive operational data from surgical elements 56222, 56224, operational data store 56203, and/or an indication of an adaptive recognition event from detector 56211. Controller 56212 may transmit data to one or more components. In examples, a controller 56212 may transmit an request for information to surgical elements 56222, 56224, a request for operational data to an operational data store 56203, a request for data to an HCP 56208 (e.g., via user interface service 56213), and/or the like.

A controller 56212 may determine an automation strategy to reduce manual tasks performed by an HCP, improve efficiency and patient safety, and/or further optimize a procedure. A controller 56212 may determine an automation strategy before and/or during a procedure (e.g., during the course of a procedure based on a request from an HCP 56208, based on a system-generated request, and/or based on an action, event, and/or the like). As part of an automation assistance strategy, a controller 56212 may indicate (e.g., via an automation assistance parameter) a first set of tasks that may be automated by surgical element 56222, 56224 during a first portion of a procedure, and/or may indicate (e.g., via a second automation assistance parameter) a second set of tasks that may be automated during a second portion of a procedure.

A controller 56212 may determine an automation strategy based on operational data and/or assistance data. In examples, controller 56212 may use operational data and/or assistance data to determine whether a task associated with a procedure may be automated (e.g., based on the availability of surgical elements 56222, 56224 and/or the like).

Assistance data may include ML models, one or more tasks associated with a procedure, a property associated with a task, and/or instructions for performing a task, as described herein. A controller 56212 may transmit operational data and/or assistance data as an input to ML models, to determine an automation strategy (e.g., to determine an automation assistance parameter as described herein). Controller 56212 may send an instruction to surgical element 56222, 56224, to automate a task associated with a procedure. An instruction may include a setpoint, an output characteristic (e.g., a power level, an RPM of a cutting tool, a position of a robotic arm, and/or the like), an indication to perform a task, and/or the like.

An automation strategy may be determined in response to receiving an indication of an adaptive recognition event (e.g., from detector 56211). For example, controller 56212 may determine a strategy based on an indication of a determined relationship and/or based on an assessment of a risk to a patient's safety (e.g., an indication that a physiological parameter of a patient 56209 has satisfied a life-threatening threshold). In examples, a controller 56212 may determine a first strategy for a procedure that automates a first portion of a procedure (e.g., a task, a plurality of tasks, and/or the like). The first strategy may include a first set of tasks that are automated by the system 56210, based on a user input from an HCP 56208 (e.g., an HCP 56208 may select to manually perform a first set of tasks while selecting to automate a second set of tasks during a procedure). The controller 56212 may indicate (e.g., may display an automation assistance strategy via user interface service 56213) that the HCP 56208 is performing a first set of tasks.

The controller 56212 may receive an indication of one or more relationships as described herein. A relationship may be determined based on, for example, data received from a surgical element 56222 and 56224. If the controller 56212 receives an indication of a relationship during the procedure (e.g., a surgical element 56222 may not be reliable and/or is not working properly), the controller 56212 may dynamically determine an automation strategy that enables the HCP 56208 to continue performing a tasks associated with the first automation strategy and/or send an indication to the HCP 56208 that a previously automated task may not be automated based on the relationship.

The controller 56212 may receive an indication that a physiological parameter has satisfied a threshold as described herein (e.g., an event). Based on a received event, the controller 56212 may determine an automation strategy (e.g., that increases the level of automation associated with a procedure), such that the HCP 56208 may direct their concentration, focus, and/or attention to a strategic portion of a procedure. As an illustrative example, if a patient's core body temperature decreases below a threshold (e.g., if a life-threatening event is detected), the controller 56212 may determine (e.g., select) an automation strategy that (e.g., fully) automates one or more tasks associated with control of an output characteristic of the ventilator and/or a heating blanket (e.g., to stabilize and/or correct a patient's core body temperature). As result of the determined automation strategy, a procedure may be performed more efficiently, because a surgeon may continue focusing their attention on removing tissue from a tumor located on a patient's heart instead of stopping the procedure, and directing their attention towards stabilizing and/or maintaining the patient's core body temperature.

The controller 56212 may determine an automation strategy based on one or more ML models. In examples, controller 56212 may transmit operational data, an indication of an event, and/or assistance data as an input to an ML model (e.g., stored on assistance data store 56214). In response, controller 56212 may receive an output from the ML model, including an automation assistance parameter (e.g., such as an instruction for a surgical element 56222 to automate a task). Controller 56212 may send data (e.g., operational data, an automation assistance parameter, an indication of an event, and/or the like) to ML model trainer 56215, to be used as training data. Data may be used to generate training data, to train one or more ML models (e.g., as part of ML model trainer 56215 as described herein).

An automation assistance parameter may indicate task(s) and/or a property associated with a task to be automated during a procedure. For example, an automation assistance parameter may indicate a task identification, a procedure, a start/end time to perform a task, a duration, a surgical element 56222, 56224 assigned to perform the task, a task priority, a task risk value, an automation level identifier, and/or the like. Controller 56212 may display (e.g., via user interface service 56213) an automation assistance parameter to an HCP 56208, send the automation assistance parameter to surgical element 56222, 56224, and/or store the automation assistance parameter (e.g., in assistance data store 56214 and/or operational data store 56203). As an illustrative example, an automation assistance parameter may include a surgical element identification (serial number, port number, model number and/or the like), an instruction (e.g., for a surgical element to automate the task), a start/stop time for a task, a setpoint, and/or an output characteristic (e.g., a power level, an RPM of a cutting tool, a position of a robotic arm, and/or the like).

In examples, an assistance parameter may include an automation level identifier. In examples, controller 56212 may instruct a surgical element 56222, 56224 to automate task(s) including an automation level identifier. In examples, the number of automated tasks increases as the automation level identifier increases. An automation level identifier may be a number associated with a group of tasks to be automated by a procedure (e.g., 1, 2, 3, 4 and/or the like, where 1 includes only a few automated tasks whereas 4 fully automates tasks associated with a procedure). In examples, an automation level identifier may include a name (e.g., assistant, partner, gopher, understudy, and/or the like).

The controller 56212 may determine a task that may be automated and then allow the user (e.g., an HCP 56208 via a user interface service 56213 to select whether to automate the task. In examples, the HCP 56208 may select to manually perform an indicated task, and/or request that the system 56210 obtain additional operational data based on execution of the manual task. In examples, the system 56210 may receive acknowledgement that a first task is successfully automated before determining a second task to automate. As an illustrative example, the system 56210 may perform suturing numerous times to develop a basic level of competency in suturing (e.g., as determined by the HCP 56208). The system 56210 may be indicate the corresponding elements of arm positioning, etc., before determining a second automated task.

The system 56210 may provide automation levels (e.g., as indicated in an automation assistance parameter) associated with supporting an HCP 56208 during a procedure based on the aspect of the surgeon. For example, for opening and closing a patient, a robotic system (e.g., a surgical element 56222) may provide a lower level of automation. However, for laparoscopic dissection within the procedure, the robotic system may provide a higher level of automation (e.g., the system 56210 may determine a second automation assistance parameter based on one or more tasks associated with a procedure), and then for stapling actions, the robotic system may provide a lower level of support.

In examples, the automation assistance parameter may indicate instructions associated with routine tasks and/or complex tasks. For example, in suturing, an automation assistance parameter may indicate simple suturing scenarios, as well as more complicated suturing scenarios based on tissue, anatomical access, and other constraints (e.g., based on operational data).

Monitoring user controlled actions in order to suggest automating the actions based on situational awareness may be described herein. In examples, the system 56210 (e.g., controller 56212) may monitor the HCP 52608 utilization, procedure steps, decisions, and/or the like, to detect a pattern of behavior to enable the system 56210 to suggest a task (e.g., minor operations) that the system 56210 may perform (e.g., may generate a surgical assistance parameter as described herein to cause surgical element 56222, 56224 to execute a task associated with a procedure). A suggested task may be performed automatically by a surgical element 56222, 56224 to relieve repetitive tasks, preparations, or setups from an HCP 56208.

In examples, the system 56210 may determine an automation assistance parameter based learning across surgeons (e.g., determining a relationship between data associated with a surgeon's actions). As an example, the system 56210 may adapt (e.g., determine relationships) based on data gathered from a right-handed and/or left-handed surgeon (e.g., the system 56210 may determine a relationship based on operational data including whether an HCP 56208 is right-handed or left-handed). By learning across multiple surgeons, the system 56210 may determine generalized items (e.g., relationships between HCPs 56208, surgical elements 56222, 56224), and/or the preferences or nuances of a specific surgeon. A task that a system 56210 may automate may be agnostic to the surgeon. In examples, one or more automation levels may be described and/or assigned based on an automation assistance parameter. In examples, a lower level of automation may be shared without creating additional risk to the patient and/or during a procedure. In examples, operational data may include surgeon specific data. To minimize conflicts and/or risks from differences in numerous surgeons, leanings may not be shared across multiple surgeons (e.g., a relationship may not be determined across one or more surgeons if the system 56210 determines that the relationship may be risk adverse to the patient and/or procedure).

As described herein, a database of prior surgeon cases to build for training a model may be included (e.g., in assistance data store, operational data store, and/or the like). Existing surgical video and/or procedures may be included and/or utilized (e.g., as part of operational data) to allow for the system 56210 to review and/or understand how to improve a response (e.g., to determine an automation assistance parameter). In examples, if a surgeon is using multiple systems (e.g., a first and second surgical element 56222, 56224), preferences and insights (e.g., operational data) from a first surgical element 56222 may be shared with a second surgical element 56224.

The controller 56212 may determine an automation assistance parameter based on, among other inputs described herein, a procedure plan, order of jobs or instruments, order of step for use of an instrument, and/or reactions to repeating issues, and/or obstacle of constraints. Examples described herein may be used to identify and/or highlight surgical element 56222, 56224, actions that may have a sequence or an event trigger, in order for the system 56210 to then start and complete the task based on its recognition of the trigger or sequence. (e.g., the system 56210 may determine/detect an adaptive recognition event based on operational data described herein).

As an illustrative example, skeletonization or direction of mesentery around the colon during a sigmoidectomy is used to free the colon from its fixation points to the body. This involves inserting a dual jaw grasping device into the semi-transparent connective tissue to form a hole, and then utilizing a spring to enlarge the hole. Once a sufficient opening is made around the arteries and/or capillaries (e.g., which are opaque and red), the device is clamped on tissue. Once clamped, energy is used to coagulate and transect the artery. An advanced visualization system (e.g., a surgical element 56222) may be used to monitor the tissues that a dual jaw system (e.g., a surgical element 56224) is interacting with. The dual jaw system may use tissue impedance to determine if the dual jaw system is interacting with tissue on the outside of the jaws or in-between the jaws. The system 56210 may monitor the procedure plan and/or instruments in use to communicate that this is a collect procedure, and that the surgeon is at the planned dissection of the mesentery steps. When the jaws are closed for insertion and there is no tissue between the jaws, energy generation may not occur automatically. When the jaws are closed on tissue in-between the jaws and the visualization system identifies an artery, the generator may automatically activate to cauterize the artery. In examples, the system 56210 may determine that if the jaws pinch connective tissue, the energy may not be activated. The operational data from the system 56210 and advanced visualization system (e.g., surgical element 56222) may provide the energy generator (e.g., surgical element 56224) with data on the tissue type which could be combined with data originating from the generator itself (e.g., tissue impedance) identifying the location of the tissue. The operational data (e.g., the tissue type, tissue impedance, tissue location data, and/or the like) may then be used to trigger the automatic activation of energy (e.g., used to generate an automation assistance parameter including an indication to trigger the activation of energy) rather than waiting for the surgeon to activate a control (e.g., via a manual process to control the activation of energy).

A system 56210 may include a user interface service 56213. User interface service 56213 may allow the system 56210 to interact with the user (e.g., an HCP 56208). User interface service 56213 may generate a GUI displayed by the system 56210 and/or by surgical element 56222, 56224. User interface service 56213 may receive data from the system 56210 and/or surgical elements 56222, 56224, and/or transmit data to the other various components of the operational environment 56200. User interface service 56213 may generate a GUI to display data from one or more components of operating environment 56200, including for example, operational data (e.g., a physiological parameter of a patient), an indication that an event has occurred, criteria associated with an event (e.g., a relationship and/or a threshold value), a description of a task, a property associated with a task, and/or the like. A user interface service 56213 may request a user input from an HCP 56208. In examples, a user interface service 56213 may display a request for information associated with a surgical element 56222, 56224 (e.g., an identification, a function, capability, an output, an input, and/or the like), a request associated with an automation strategy (e.g., to approve a determined automation strategy, to amend an automation strategy, to select an automation assistance parameter, and/or the like), a request for a task and/or procedure to be performed, a request for information related to a patient 56209, and/or the like.

A system 56210 may include assistance data store 56214. Assistance data store 56214 may store operational data, training data, an automation assistance parameter, ML models, and/or the like as described herein. In examples, the system 56210 may transmit data to the assistance data store 56214 in response to a user input (e.g., via user interface service 56213) from an HCP 56208.

A system 56210 may include ML model trainer 56215. ML model trainer 56215 may receive training data from controller 56212. Training data may include operational data, an automation assistance parameter, an indication of an event, assistance data, and/or the like. In response to receiving training data, ML model trainer 56215 may train an ML model to, among other things, detect an event, determine one or more tasks that may be automated by a surgical element 56222, 56224, and/or determine an automation assistance parameter.

ML model trainer 56215 may train an ML model to detect an adaptive recognition event. An ML model may be used to determine whether a physiological parameter satisfies a threshold and/or determine a relationship between surgical elements 56222, 56224. As an illustrative example, an ML model may receive an indication of a procedure, and/or an identification of a first surgical element 56222 (e.g., a heating blanket) and a second surgical element 56224 (e.g., a ventilator). The ML model may determine a relationship for example, that based on data indicating that if/when ventilator increases the tidal volume of air to a patient, a heating blanket's power output is increased to maintain the patient's core body temperature. An ML model may transmit an indication of a relationship to the controller 56212 (e.g., and/or the controller 56212 may adjust an automation strategy based on the relationship). For example, the controller 56212 may, in response to receiving a determined relationship from an ML model, instruct a surgical element 56222 to automate one or more tasks based on the relationship.

ML model trainer 56215 may train an ML model to determine one or more tasks that may be automated by a surgical element 56222, 56224. As an illustrative example, an ML model may receive operational data (e.g., historical data and/or real-time data during a procedure) from surgical elements 56222, 56224, an HCP 56208, assistance data store 56214, and/or the like. The operational data may include, for example, position data associated with the movement of a robotic arm during a task (e.g., position data output from a surgical element 56222 as controlled by an HCP 56208). The ML model may determine, based on the received position data, instructions that may cause the robotic arm to automatically perform one or more movements associated with a task. The instructions may be stored in, for example, assistance data store 56214. The instructions may be associated with an automation assistance parameter (e.g., the automation assistance parameter may include an indication to cause a surgical element 56222 to execute a task automatically).

ML model trainer 56215 may train an ML model to determine an automation assistance parameter. As described herein, an automation assistance parameter may indicate task(s) to be automated during a procedure and/or one or more properties associated with a task (e.g., a task identification, a procedure, a start/end time to perform a task, a duration, a surgical element 56222, 56224 to perform the task, a task priority, a task risk value, an automation level identifier, and/or the like).

An operational environment 56200 may include surgical element 56222 and/or surgical element 56224. Although two surgical elements are depicted as part of operational environment 56200, this is not meant to be limiting as multiple surgical elements may operate and/or communicate with one or more components of the operational environment 56200. Surgical element 56222, 56224, may include (not illustrated in FIG. 6) sensors, instrumentation, and/or tools as described with reference to 20282 of FIG. 5. Surgical element 56222, 56224, may be operated by an HCP 56208 and/or may operate autonomously (e.g., based on an automation strategy) to execute one or more tasks associated with a procedure. As an illustrative example, a surgical element 56222, 56224, may include a robotic surgical system, a navigation system, smart imaging systems, endoscopic and/or laparoscopic systems, harmonic scalpels, anesthesia machines, patient monitoring systems (pulse oximeters, blood pressure monitors, EKG monitors, EEG monitors, and/or the like), energy devices (e.g., electrosurgical units, laser surgery systems, and/or the like), infusion pumps, and/or the like.

In examples, surgical element 56222, 56224 may be wirelessly connected and/or physically connected (e.g., wired) to a network 56201, a system 56210, and/or another surgical element 56224. A surgical element 56222, 56224 may generate operational data. A surgical element 56222, 56224 may transmit operational data to a system 56210 (e.g., to determine an automation strategy), an operational data store 56203, and/or the like. For example, a surgical element 56222, 56224 may send/receive environmental data, a setpoint, output characteristic, measured variable, communication data, and/or user data as described herein.

As an illustrative example, a surgical element 56222 may be a heating blanket. The heating blanket may include a temperature measurement device and/or a heating element. The heating blanket may be configured to energize the heating element, to maintain a patient's core body temperature during open heart surgery, based on a user input (e.g., in an open loop) and/or based on an automation assistance parameter (e.g., in a closed loop). The heating blanket may transmit a core body temperature measurement (e.g., as operational data) to the system 56210. The surgical element 56222 may receive an automation assistance parameter (e.g., if the system 56210 determines that an event has occurred, such as a determined relationship and/or that the core body temperature has satisfied a life-threatening threshold). The automation assistance parameter may include a setpoint for the heating blanket, an output power level for the heating blanket, a duration to maintain a power level, an indication that the heating blanket is to operate automatically, and/or the like. Advantageously, the heating blanket may receive an instruction to automatically control a patient's core body temperature based on the occurrence of an event, such that the HCP 56208 may direct their concentration, focus, and/or attention to a strategic portion of a procedure.

An operational environment 56200 may include an operational data store 56203. Operational data store 56203 may be an external data store (e.g., located on a third party server and/or the like). Operational data store 56203 may store operational data, training data, an automation assistance parameter, assistance data, and/or the like. In examples, the system 56210 may transmit data to the operational data store 56203 in response to a user input (e.g., via user interface service 56213) from an HCP 56208. While the operational data store 56203 is depicted as being external to the system 56210, this is not meant to be limiting. In examples, operational data store 56203 may be a database residing on a surgical element 56222, 56224. In examples, data associated with operational data store 56203 may be transmitted from surgical element 56222, 56224 to the system 56210 (e.g., detector 56211, controller 56212, user interface service 56213, assistance data store 56214, and/or ML model trainer 56215), via network 56201.

An operational environment 56200 may include a network 56201. The network 56201 may include one or more communications networks, such as the Internet. The network 56201 may be any combination of local area network ("LAN") and/or a wireless area network ("WAN") or the like. Accordingly, various components of the operational environment 56200, including the system 56210, surgical element 56222, 56224, and/or operational data store 56203 may communicate with one another directly or indirectly via any appropriate communications links and/or networks, such as network 56201 (e.g., one or more communications links, one or more computer networks, one or more wired or wireless connections, the Internet, and/or the like).

### Example Flow Diagram for Determining to Automate Task(s)

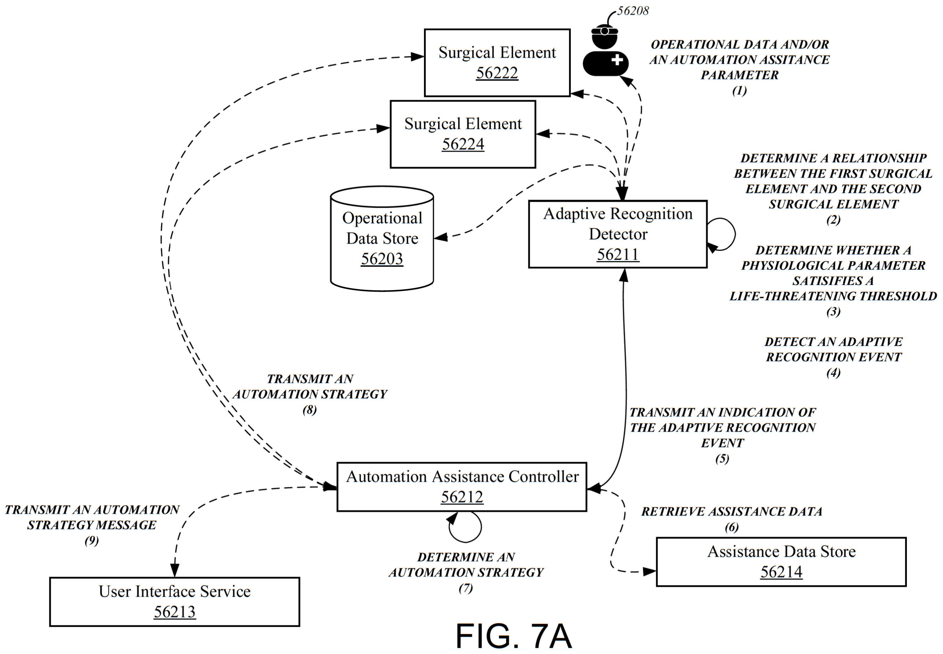

FIG. 7A is a flow diagram illustrating example operations performed by the components of the operating environment 56200 of FIG. 6, to determine whether to automate one or more tasks based on inputs from surgical elements 56222, 56224, an HCP 56208, and/or operational data store 56203.

As illustrated in FIG. 7A, an HCP 56208, surgical element 56222, 56224, and/or operational data store 56203 transmits operational data, and/or an automation assistance parameter to a detector 56211 at (1). As described herein, operational data may include past and/or present information associated with an operational environment 56200 such as data generated based on one or more procedures (e.g., past and/or present procedures). In examples, operational data may include: a physiological parameter of a patient (e.g., a patient's heart rate, blood pressure, SpO2, core body temperature, and/or the like); environmental data (e.g., a number of HCPs in an operating room, historical records of an HCP 56208 performing a procedure, position data associated with the movement an HCP 56208 during a procedure, data associated with a hospital such as a quantity of procedures performed and/or an outcome of procedure(s) or function(s) associated with surgical elements 56222, 56224 available during a procedure, and/or the like); data associated with surgical elements 56222, 56224 (e.g., a setpoint, output characteristic, measured variable, communication data, position data, and/or the like), user data (e.g., data generated based on a user input by an HCP 56208 via user interface service 56213), and/or the like.

An automation assistance parameter may include a surgical element identification (serial number, port number, model number and/or the like), an instruction (e.g., for a surgical element to automate the task and/or instructions associated with performing a task), a start/stop time for a task, a setpoint, and/or an output characteristic (e.g., a power level, an RPM of a cutting tool, a position of a robotic arm, and/or the like).

As an illustrative example, an HCP 56208 may transmit, via user interface service 56213, an automation assistance parameter indicating that a surgical element 56222 is to maintain a core body temperature of a patient during a portion of a procedure (e.g., the surgical element 56222 is to operate in an automatic mode while the surgeon is removing tissue from a patient's heart during open heart surgery).

Once the detector 56211 receives the operational data and/or automation assistance parameter, the detector 56211 may determine a relationship between the first surgical element and the second surgical element at (2), and/or determine whether a physiological parameter satisfies a life-threatening threshold at (3) (e.g., determine an adaptive recognition event). For example, detector 56211 may determine a relationship between a first surgical element 56222, a second surgical element 56224, a patient 56209, and/or an HCP 56208. Detector 56211 may generate a look-up-table including one or more determined relationships and/or determine a relationship based on analysis of a look-up-table (e.g., by referencing the look-up table based on a selected procedure by an HCP 56208). As an illustrative example, a detector 56211 may determine a relationship (e.g., based on an analysis of operational data) between a core body temperature (e.g., a setpoint measured by a surgical element 56222), a ventilator (e.g., surgical element 56224), and/or a heating blanket (e.g., one or more additional surgical elements not illustrated as part of operational environment 56200). The detector 56211 may determine the relationship based on data indicating that if ventilator increases the tidal volume of air to a patient 56209, a heating blanket's power output may be increased to maintain the patient's core body temperature.

A detector 56211 may determine that an event has occurred based on whether a physiological parameter satisfies a threshold (e.g., a life-threatening threshold). A threshold may be determined by the system 56210, determined by an HCP 56208 (e.g., received via a user input), determined by an ML model, determined by detector 56211, and/or based on operational data associated with the patient 56209. In examples, a detector 56211 may determine that an event has occurred based on a condition that one, two, three, four and/or more physiological parameters satisfy a threshold. A detector 56211 may determine one or more aspects of a threshold based on operational data (e.g., data associated with a procedure, aspects of the surgical element(s) 56222, 56224 available during the procedure, an HCP 56208, and/or a patient 56209). Aspects of a threshold may include a threshold type (e.g., heart rate, SpO2, and/or the like), a threshold value (e.g., 90 degrees Fahrenheit for a core body temperature, a range for a heart rate, and/or the like), and/or the quantity of threshold(s) (1, 2, 3, 4, etc.) may vary for a procedure.

Based on the determination of a relationship between the first surgical element 56222 and the second surgical element 56224 (e.g., and/or the HCP 56208, patient 56209, and/or the like) at (2), or based on a determination that a physiological parameter satisfies a threshold at (3), detector 56211 may determine that an adaptive recognition event has occurred at (4).

After the detector 56211 has determined that an adaptive recognition event has occurred, the detector 56211 may transmit an indication of the adaptive recognition event to the controller 56212 at (5). The indication of an adaptive recognition event may include information associated with the determined relationship and/or the physiological parameter of the patient 56209 that satisfied a threshold as described herein.

Once the controller 56212 receives the indication of the adaptive recognition event, the controller may retrieve assistance data from assistance data store 56214 at (6). As described herein, assistance data may include ML models, one or more tasks associated with a procedure, a property associated with a task, and/or instructions for performing a task. Optionally, the controller 56212 may transmit operational data, an indication of the adaptive recognition event, and/or an automation assistance parameter to assistance data store 56214, as an input to ML models (ML models stored in assistance data store), to determine an automation strategy (e.g., to determine an automation assistance parameter as described herein).

Controller 56212 may determine an automation strategy at (7). An automation assistance strategy may be determined in response to receiving an indication of an adaptive recognition event (e.g., from detector 56211), an automation assistance parameter, and/or operational data. For example, controller 56212 may determine a strategy (e.g., including an automation assistance parameter) based on an indication of a determined relationship and/or based on an assessment of a risk to a patient's safety (e.g., an indication that a physiological parameter of a patient 56209 has satisfied a life-threatening threshold). In examples, a controller 56212 may determine a first strategy for a procedure that automates a first portion of a procedure (e.g., a task, a plurality of tasks, and/or the like). The first strategy may include a first set of tasks that may be automated by the system 56210 based on a user input from an HCP 56208 (e.g., an HCP 56208 may select to manually perform a first set of tasks while selecting to automate a second set of tasks during a procedure).

The controller 56212 may transmit the determined automation strategy (e.g., an automation assistance parameter) to surgical elements 56222, 56224 at (8). As described herein, the transmitted strategy may include an automation assistance parameter. The automation assistance parameter may provide instructions for performing a task associated with a procedure. In examples, an automation assistance parameter may indicate that a surgical element 56222 will fully automate a procedure. In examples, an automation assistance parameter may include a setpoint, an output characteristic (e.g., a power level, an RPM of a cutting tool, a position of a robotic arm, and/or the like), an indication/instruction to perform a task, and/or the like. The automation assistance parameter may indicate task(s) and/or a property associated with a task to be automated during a procedure. For example, an automation assistance parameter may indicate a task identification, a procedure, a start/end time to perform a task, a duration, a surgical element 56222, 56224 assigned to perform the task, a task priority, a task risk value, an automation level identifier, and/or the like.

The controller 56212 may transmit an automation strategy message to the user interface service 56213 at (9). The automation strategy message may indicate and/or instruct the HCP 56208 to perform a first set of tasks and indicate that the surgical element 56222 may perform a second set of tasks. Optionally, the user interface service 56213 may receive, in response to displaying the automation strategy message to the HCP 56208, a user input authorizing the selected automation tasks (e.g., to be performed manually by the HCP 56208 and/or performed automatically by the surgical element 56222, 56224).

### Example Flow Diagram for Training an ML Model

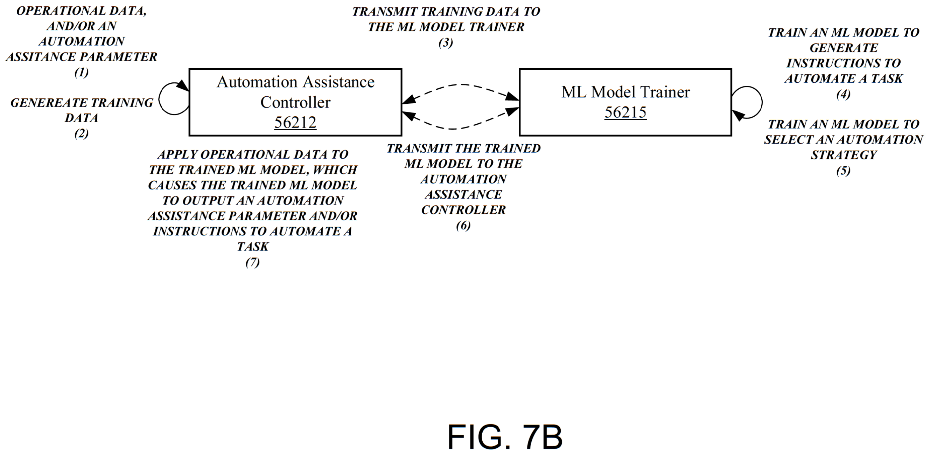

FIG. 7B is a flow diagram illustrating example operations performed by the components of the operating environment 56200 of FIG. 6, to train an ML model to determine an automation assistance parameter and/or an automation strategy during a procedure based on inputs from an HCP 56208, surgical elements 56222, 56224, and/or an operational data store 56203.

As illustrated in FIG. 7B, controller 56212 may obtain operational data and/or an automation assistance parameter from an HCP 56208, a surgical element 56222, 56224, and/or operational data store 56203) at (1). As described herein, controller 56212 may receive operational data and/or an automation assistance parameter from surgical elements, 56222, 56224, operational data store 56203, from an HCP 56208, and/or the like.

The controller may generate training data at (2). Training data may be generated based on the operational data and/or automation assistance parameter. Optionally, training data may include operational data, an automation assistance parameter, an indication of an event (e.g., an adaptive recognition event), assistance data, and/or the like. In response to receiving training data, ML model trainer 56215 may train an ML model to, among other things, detect an event, determine one or more tasks that may be automated by a surgical element 56222, 56224, and/or determine an automation assistance parameter.

Once the training data has been generated, controller 56212 may transmit the training data to the ML model trainer 56215 at (3).

The ML model trainer 56215 may receive the training data, and train a model to generate instructions to automate a task at (4). As described herein, ML model trainer 56215 may train an ML model to determine one or more tasks that may be automated by a surgical element 56222, 56224. As an illustrative example, an ML model trainer 56215 may receive training data including position data associated with the movement of a robotic arm during a task (e.g., position data output from a surgical element 56222 as controlled by an HCP 56208). The ML model trainer 56215 may train an ML model to determine, based on the received position data, instructions that may cause a robotic arm to automatically perform one or more movements associated with a task.

The ML model trainer 56215 may receive the training data, and train an ML model to select an automation strategy at (5). For example, ML model trainer 56215 may train an ML model to determine an automation assistance parameter as part of an automation strategy. As described herein, an automation assistance parameter may indicate task(s) to be automated during a procedure and/or one or more properties associated with a task (e.g., a task identification, a procedure, a start/end time to perform a task, a duration, a surgical element 56222, 56224 to perform the task, a task priority, a task risk value, an automation level identifier, and/or the like).

Optionally, ML model trainer 56215 may train an ML model to detect an adaptive recognition event based on training data. An ML model may be used to determine whether a physiological parameter satisfies a threshold and/or determine a relationship between surgical elements 56222, 56224. As an illustrative example, an ML model may be trained to receive an indication of a procedure, and/or an identification of a first surgical element 56222 (e.g., a heating blanket) and a second surgical element 56224 (e.g., a ventilator). The ML model may be trained to determine a relationship for example, based on data indicating that if ventilator increases the tidal volume of air to a patient, a heating blanket's power output may be increased to maintain the patient's core body temperature. The output of a first ML model (e.g., trained to detect an adaptive recognition event) may be sent to a second ML model trained to determine an automation strategy. For example, ML model trainer 56215 may train a second ML model to receive an indication of an adaptive recognition event, and instruct a surgical element 56222 to automate one or more tasks based on the detected event.

After ML model trainer 56215 has trained an ML model, ML model trainer 56215 may transmit the trained ML model to the controller 56212 at (6). Additionally and/or optionally, ML model trainer 56215 may transmit the trained ML model to another component of the operational environment 56200 (e.g., assistance data store 56214, operational data store 56203, and/or the like).

Once the controller 56212 receives the trained ML model from ML model trainer 56215, the controller 56212 may apply operational data, assistance data, and/or the like, as an input to the trained ML model, which causes the trained ML model to output an automation assistance parameter and/or instructions to automate a task at (7).

### Example Aspects Related to an Adaptive Recognition Detector Routine

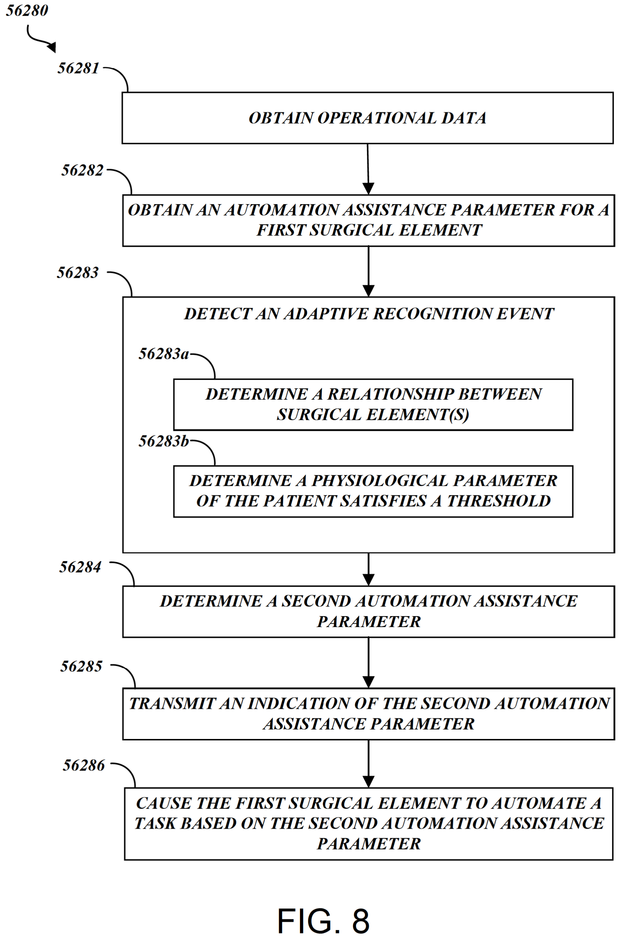

FIG. 8 is a flow chart of an adaptive recognition detector routine 56280 illustratively implemented by a system 56210. As an example the system 56210 of FIG. 6 (e.g., detector 56211 and/or controller 56212) may be configured to execute the adaptive recognition detector routine 56280. The routine 56280 begins at block 56281.

At block 56281, a system 56210 may obtain operational data. As described herein, a system 56210 may obtain operational data from surgical elements 56222, 56224, an HCP 56208, and/or an operational data store 56203. Operational data may include past and/or present information associated with an operational environment 56200. In examples, operational data may include a physiological parameter of a patient, environmental data, data associated with surgical elements 56222, 56224, user data and/or the like.

At block 56282, a system 56210 may obtain an automation assistance parameter for a first surgical element 56222. The system 56210 may obtain the automation assistance parameter from a surgical element 56222, 56224, from an HCP 56208 (e.g., via user interface service 56213), from operational data store 56203, from assistance data store 56214, and/or from another component of the operational environment 56200. In examples, the system 56210 may determine a first automation assistance parameter based on operational data. As described herein, an automation assistance parameter may indicate task(s) and/or a property associated with a task to be automated during a procedure. For example, an automation assistance parameter may indicate a task identification, a procedure, a start/end time to perform a task, a duration, a surgical element 56222, 56224 assigned to perform the task, a task priority, a task risk value, an automation level identifier, and/or the like. In examples, controller 56212 may display (e.g., via user interface service 56213) an automation assistance parameter to an HCP 56208, send the automation assistance parameter to surgical element 56222, 56224, and/or store the automation assistance parameter (e.g., in assistance data store 56214 and/or operational data store 56203).

As an illustrative example, an automation assistance parameter may include a surgical element identification (serial number, port number, model number and/or the like), an instruction (e.g., for a surgical element to automate the task), a start/stop time for a task, a setpoint, and/or an output characteristic (e.g., a power level, an RPM of a cutting tool, a position of a robotic arm, and/or the like).

In examples, an assistance parameter may include an automation level identifier. In examples, controller 56212 may instruct a surgical element 56222, 56224 to automate task(s) including an automation level identifier. In examples, the number of automated tasks increases as the automation level identifier increases.

At block 56283, the system 56210 may detect an adaptive recognition event. As described herein, an adaptive recognition event may include a determination of a relationship and/or a determination that a physiological parameter satisfies a threshold (e.g., as determined by the detector 56211).

Optionally at block 56283a, the system 56210 (e.g., detector 56211) may determine a relationship between a first surgical element 56222, a second surgical element 56224, a patient 56209, and/or an HCP 56208. Detector 56211 may determine a relationship based on operational data (e.g., data generated during a procedure and/or based on historical operational data). The system 56210 may determine a relationship, for example, in response to a user input (e.g., a user input from an HCP 56208 via a user interface service 56213). The system 56210 may generate a look-up-table including one or more determined relationships and/or determine a relationship based on analysis of a look-up-table (e.g., by referencing the look-up table based on a selected procedure by an HCP 56208).

As an illustrative example, a detector 56211 may determine a relationship (e.g., based on an analysis of operational data) between a core body temperature (e.g., a setpoint measured by a surgical element 56222), a ventilator (e.g., surgical element 56224), and/or a heating blanket (e.g., one or more additional surgical elements not illustrated as part of operational environment 56200). The detector 56211 may determine a relationship, for example, based on data indicating that if/when ventilator increases the tidal volume of air to a patient 56209, a heating blanket's power output increases to maintain the patient's core body temperature.

Optionally at block 56283b, the system 56210 (e.g., detector 56211) may determine that a physiological parameter satisfies a threshold (e.g., as determined by detector 56211). As described herein, the system 56210 may receive data associated with a physiological parameter of a patient from surgical element 56222, 56224, and compare the data to a threshold. A threshold may be determined by the system 56210, determined by an HCP 56208 (e.g., received via a user input), determined by an ML model, and/or based on operational data associated with the patient 56209. In examples, the system 56210 may determine that an event has occurred based on a condition that one, two, three, four and/or more physiological parameters satisfy a threshold.

The system 56210 may determine one or more aspects of a threshold based on operational data (e.g., data associated with a procedure, data from a surgical element 56222, 56224 available during the procedure, an HCP 56208, and/or a patient 56209) and/or an automation assistance parameter. In examples, aspects of a threshold may include a threshold type (e.g., heart rate, SpO2, and/or the like), a threshold value (e.g., 90 degrees Fahrenheit for a core body temperature, a range for a heart rate, and/or the like), and/or the quantity of threshold(s) (1, 2, 3, 4, etc.) may vary for a procedure.

At block 56284, the system 56210 may determine a second automation assistance parameter. The second automation assistance parameter may be determined based on operational data, the adaptive recognition event, assistance data, a user input (e.g., by an HCP 56208 via user interface service 56213), and/or based on an ML model (e.g., as trained by ML model trainer 56215). The automation assistance parameter may include instructions for performing a task associated with a procedure. In examples, an automation strategy may indicate, to a surgical element 56222, to fully automate a procedure. In examples, the automation assistance parameter may include a setpoint, an output characteristic (e.g., a power level, an RPM of a cutting tool, a position of a robotic arm, and/or the like), an indication/instruction to perform a task, and/or the like. An automation assistance parameter may indicate task(s) and/or a property associated with a task to be automated during a procedure. For example, an automation assistance parameter may indicate a task identification, a procedure, a start/end time to perform a task, a duration, a surgical element 56222, 56224 assigned to perform the task, a task priority, a task risk value, an automation level identifier, and/or the like.

In examples, a controller 56212 may determine a first automation assistance parameter for a procedure that automates a first portion of a procedure (e.g., a task, a plurality of tasks, and/or the like). The first parameter may include a first set of tasks that may be automated by the system 56210 based on a user input from an HCP 56208 (e.g., an HCP 56208 may select to manually perform a first set of tasks while selecting to automate a second set of tasks during a procedure).

Optionally, the automation assistance parameter may be determined by an ML model. As described herein, ML model trainer 56215 may receive the training data, and train an ML model to select an automation strategy (e.g., determine a second automation assistance parameter). The system 56210 may receive a trained ML model from ML model trainer 56215 and apply operational data, assistance data, and/or the like, to the trained ML model. The trained ML model may output an automation assistance parameter and/or instructions to automate a task.

At block 56285, the system 56210 may transmit an indication of the second automation assistance parameter to surgical element 56222, 56224. The indication of the second automation assistance parameter may include, as described herein, a task identification, a procedure, a start/end time to perform a task, a duration, a surgical element 56222, 56224 assigned to perform the task, a task priority, a task risk value, an automation level identifier, and/or the like.

As an illustrative example, a surgical element 56222 may be a heating blanket. The heating blanket may include a temperature measurement device and/or a heating element. The heating blanket may be configured to energize the heating element, to maintain a patient's core body temperature during open heart surgery, based on a user input (e.g., in an open loop) and/or based on an automation assistance parameter (e.g., in a closed loop). The surgical element 56222 may receive an automation assistance parameter (e.g., if the system 56210 determines that an event has occurred, such as a determined relationship and/or that the core body temperature has satisfied a life-threatening threshold). The automation assistance parameter may indicate a setpoint for the heating blanket, an output power level for the heating blanket, a duration to maintain a power level, an indication that the heating blanket is to operate automatically, and/or the like.

Optionally, the system 56210 may transmit an indication of the automation assistance parameter and/or an indication of the adaptive recognition event to the HCP 56208 (e.g., via user interface service 56213).

At block 56286, the system 56210 may cause the surgical element 56222, 56224, to automate a task based on the second automation assistance parameter. In examples, the surgical element 56222, 56224 may receive an automation assistance parameter that causes the surgical element 56222, 56224 to operate in a fully automatic mode. As an illustrative example, a surgical element 56222 (e.g., a smoke evacuation component) may receive an instruction to automatically evacuate smoke, fluid, and/or particles generated by the application of therapeutic energy from a second surgical element 56224 (e.g., operated by an HCP 56208).

An illustrative example described herein may provide an (e.g., bounded fully) autonomous operation of a first system (e.g., a first surgical element) in cooperation with an operation of another system controlled directly by the surgeon (e.g., a second surgical element).

A procedure may include an ablative remodeling of the heart to treat AFIB. An automatic RF catheter motion control system (e.g., a second surgical element controlled by the surgeon) may ablate a predefined area of tissue, with predefined depth, pressure or power intensity levels. The surgeon may provide the area of intended treatment after mapping of the heart and/or provide the depth of the ablation desired.

The system (e.g., system 56210) may display a mapped path of the catheter motion and may receive confirmation that a robot (e.g., a first surgical element) sweeps the catheter through the path maintaining the pressure and/or energy balance while compensating for the movement of the heart in the process. In examples, bounding of the automation (e.g., bounding motions of the surgeon) enables the smart system (e.g., 56210) to actively interact with and support surgeon controlled motions of instruments while including some limits to the motion to prevent the cooperative or antagonistic actions or forces from causing collateral damage, or inadvertently colliding with nearby structures (e.g., the system 56210 may determine an automation assistance parameter to bound movement of the robot that are requested by the surgeon).

To create the sufficient contact and/or pressure for a moving ablation system to cause the cautery and/or ablation (e.g., which is dependent on pressure, power and mode of energy) the user (e.g., surgeon) may control an ablative electrode. Using the ablative electrode, the user may define the electrode's path and/or pushing force capability (e.g., to create the needed energy density to produce the sufficient tissue welding or tissue death that the assisting system (e.g., the robot) would have to provide) in order to maintain the needed energy density. In examples, the heart may be a sensitive and interconnected structure that could easily be injured inadvertently if too much force or too much differential motion is applied. The robot may monitor the surgical image of the system 56210 for adjacent structures and/or may monitor its own applied forces relative to the force applied from the user. The sum of the forces relative to the fixation of the heart to the surrounding anatomy may be limited to the applicable force and motions (e.g., the robot may, based on the surgeons movement of an electrode, apply more and/or less force to maintain contact during tissue modification, resulting in an operational environment 56200 having less and/or more automation, depending on the actions of the surgeon).

### Example Aspects Related to Determining to Automation Strategy

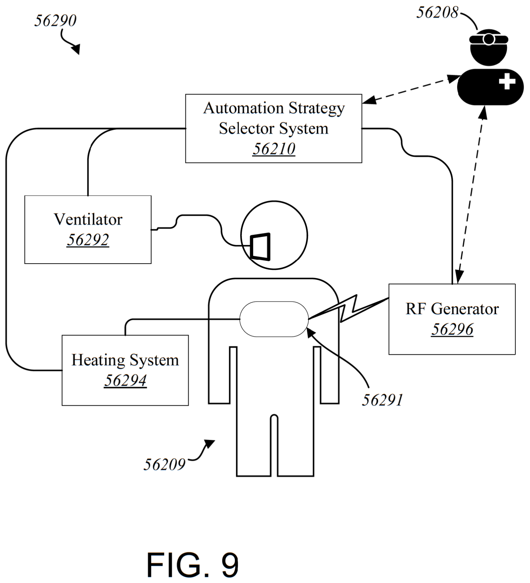

FIG. 9 is an illustrative example of an operational environment 56290 configured to determine an automation strategy. The operational environment 56290 may include a procedure for cardiac atrial fibrillation (AFIB) nerve ablation 56291, to eliminate irregular heartbeat using RF ablation. A smart ventilator 56292 (e.g., a first surgical element) may have a preset tidal volume and/or O2 supplementation percentage from an initial surgeon setup (e.g., as set by an HCP 56208). A smart patient heating system 56294 (e.g., a second surgical element) may have a preset thermal transfer rate to the patient to create a thermoneutral condition which is slightly hypothermic (34-33 C).

The smart ventilator 56292 may be set to a closed loop control using a finger based transcutaneous oxygen sensor to relate O2 blood gases to the O2 supplementation rate. The heating system 56294 may be closed loop (e.g., operating based on a first automation assistance parameter) based on the core body temperature measurement of the patient 56209 (e.g., as determined by heating system 56294). To mitigate potential collateral thermal damage from the RF generator 56296 (e.g., an RF monopolar catheter) during the ablative remodeling local organ, cooling may be used on the heart at a controlled cooling thermal load (e.g., by the heating system 56294) that is closed loop controlled by the energy density and activation timing of the RF generator 56296.

The ventilator 56292 may monitor the outgassing CO2 which enables the system 56210 to measure the exhaust CO2 percentage in each controlled breath of the patient 56209. As the procedure progresses, sedation and mild hypothermia may reduce the patient's metabolism of O2. A CO2 to O2 measurement may drift apart (e.g., as measured by the ventilator 56292 and/or another surgical element). The system 56210 may detect an adaptive recognition event if/when at least one measurement (CO2 to O2 and/or core body temperature) satisfies a threshold (e.g., the physiological parameter of the patient satisfies a life threatening threshold). The system 56210 may, based on the core body temperature, the RF generated energy, and/or the heating system 56294 setpoint (e.g., operational data), generate an automation strategy including an automation assistance parameter. The system 56210 may transmit the automation assistance parameter to the ventilator 56292, including an indication that one of the two measurements is out of synchronization, an instruction to automatically decrease an O2 supplementation level, and/or an instruction to automatically increase a tidal volume. In general, a CO2 to O2 measurement as used herein can optionally be, or comprise, a CO2 to 02 ratio.

Advantageously, the system 56210 may determine an automation strategy based on operational data and/or an adaptive recognition event associated with the ventilator 56292, heating system 56294, and/or RF generator 56296, to automatically instruct the ventilator 56292 to control O2 and/or the tidal volume, and allow the HCP 56208 to continue ablative remodeling of the local organ without delay and/or interruption to the procedure. The system 56210 determines an automation strategy to efficiently reduce procedure times rather than the conventional methods of transmitting a request to an HCP 56208, and waiting for either an acknowledgement from the HCP 56208 (e.g., for the surgical element to automatically adjust an O2 supplementation level) and/or a manual adjustment by the HCP 56208 of the O2 supplementation level.
The following list of numbered Examples forms part of the present disclosure:
1. A system to adaptively recognize an automation strategy during a procedure, the system comprising:
   a processor configured to:
   obtain operational data for a first surgical element and a second surgical element, wherein the operational data comprises an indication of a physiological parameter of a patient during the procedure, and a plurality of automated tasks associated with the procedure;
   determine an automation assistance parameter for the first surgical element, wherein the automation assistance parameter is associated with a set of automated tasks from the plurality of automated tasks that are performed by the first surgical element during the procedure;
   detect an adaptive recognition event, wherein the adaptive recognition event is at least one of:
      a relationship between the first surgical element and the second surgical element; or
      a determination that the physiological parameter of the patient satisfies a life-threatening threshold;
   based on the adaptive recognition event, select a second automation assistance parameter for the first surgical element, wherein the second automation assistance parameter indicates a second set of automated tasks that are performed by the first surgical element during the procedure;
   transmit an indication of the second automation assistance parameter to the first surgical element; and
   cause the first surgical element to perform the second set of automated tasks based on the second automation assistance parameter.
2. The system of Example 1, wherein:
   the first surgical element is a ventilator;
   the second surgical element is a heating device;
   the physiological parameter of a patient is a CO2 percentage from the ventilator or a core body temperature from the heating device;
   the procedure of the patient comprises a cardiac atrial fibrillation (AFIB) nerve ablation;
   the adaptive recognition event comprises a determination that a CO2 to O2 or a core body temperature of the patient satisfies the life-threatening threshold; and
   wherein the second automation assistance parameter comprises an instruction to decrease an O2 supplementation level of the ventilator or an instruction to increase a tidal volume of the ventilator.
3. The system of Example 1, wherein the physiological parameter of the patient satisfies a life-threatening threshold if the first surgical element or the second surgical element determines that: a heart rate exceeds an operational window of 40-120 beats per minute, an oxygen saturation of the patient decreases below 90%, or a core body temperature of the patient is less than 89 degrees Fahrenheit.
4. The system of Example 1, wherein the adaptive recognition event comprises the determination that the physiological parameter of the patient satisfies the life-threatening threshold, and wherein the selected second automation assistance parameter indicates that the second set of automated tasks comprises the plurality of automated tasks.
5. The system of Example 1, wherein the adaptive recognition event comprises the relationship between the first surgical element and the second surgical element, and wherein the selected second automation assistance parameter further comprises an instruction to perform the second set of automated tasks in response to a user input.
6. The system of Example 5, wherein the processor is further configured to:
   send an automation strategy message to a user, wherein the automation strategy message comprises a request to perform the second set of automated tasks; and
   receive a user input comprising an instruction to perform at least one automated task of the second set of automated tasks.
7. The system of Example 1, wherein the processor is further configured to:
   receive position data associated with a movement of a user during the procedure and control data associated with the first surgical element and the second surgical element; and
   determine the relationship between the first surgical element and the second surgical element is based on the position data, the operational data, and the physiological parameter of the patient.
8. The system of Example 1, wherein the processor is further configured to:
   based on the adaptive recognition event, select an automation assistance parameter for the second surgical element, wherein the automation assistance parameter for the second surgical element indicates a set of automated tasks to be performed by the second surgical element during the procedure;
   transmit, to the second surgical element, an indication of the automation assistance parameter for the second surgical element; and
   cause the second surgical element to perform the set of automated tasks based on the automation assistance parameter for the second surgical element.
9. A method for adaptively recognizing an automation strategy during a procedure, the method comprising:
   obtaining operational data for a first surgical element and a second surgical element, wherein the operational data comprises an indication of a physiological parameter of a patient during the procedure, and a plurality of automated tasks associated with the procedure;
   determining an automation assistance parameter for the first surgical element, wherein the automation assistance parameter is associated with a set of automated tasks from the plurality of automated tasks that are performed by the first surgical element during the procedure;
   detecting an adaptive recognition event, wherein the adaptive recognition event is at least one of:
      a relationship between the first surgical element and the second surgical element; or
      a determination that the physiological parameter of the patient satisfies a life-threatening threshold;
   based on the adaptive recognition event, selecting a second automation assistance parameter for the first surgical element, wherein the second automation assistance parameter indicates a second set of automated tasks that are performed by the first surgical element during the procedure;
   transmitting an indication of the second automation assistance parameter to the first surgical element; and
   causing the first surgical element to perform the second set of automated tasks based on the second automation assistance parameter.
10. The method of Example 9, wherein:
   the first surgical element is a ventilator;
   the second surgical element is a heating device;
   the physiological parameter of a patient is a CO2 percentage from the ventilator or a core body temperature from the heating device;
   the procedure of the patient comprises a cardiac atrial fibrillation (AFIB) nerve ablation;
   the adaptive recognition event comprises a determination that a CO2 to O2 or a core body temperature of the patient satisfies the life-threatening threshold; and
   wherein the second automation assistance parameter comprises an instruction to decrease an O2 supplementation level of the ventilator or an instruction to increase a tidal volume of the ventilator.
11. The method of Example 9, wherein the adaptive recognition event comprises the determination that the physiological parameter of the patient satisfies the life-threatening threshold, and wherein the selected second automation assistance parameter indicates that the second set of automated tasks comprises the plurality of automated tasks.
12. The method of Example 9, wherein the adaptive recognition event comprises the relationship between the first surgical element and the second surgical element, and wherein the selected second automation assistance parameter comprises an instruction to perform the second set of automated tasks in response to a user input.
13. The method of Example 12, wherein the method further comprises:
   sending an automation strategy message to a user, wherein the automation strategy message comprises a request to perform the second set of automated tasks; and
   receiving a user input comprising an instruction to perform at least one automated task of the second set of automated tasks.
14. The method of Example 9, wherein the method further comprises:
   receiving position data associated with a movement of a user during the procedure and control data associated with the first surgical element and the second surgical element; and
   determining the relationship between the first surgical element and the second surgical element based on the position data, the operational data, and the physiological parameter of the patient.
15. The method of Example 9, wherein the method further comprises:
   based on the adaptive recognition event, selecting an automation assistance parameter for the second surgical element, wherein the automation assistance parameter for the second surgical element indicates a set of automated tasks to be performed by the second surgical element during the procedure;
   transmitting, to the second surgical element, an indication of the automation assistance parameter for the second surgical element; and
   causing the second surgical element to perform the set of automated tasks based on the automation assistance parameter for the second surgical element.
16. A system to adaptively recognize an automation strategy, the system comprising:
   a processor configured to:
      obtain operational data for a first surgical element and a second surgical element, wherein the operational data comprises an indication of a physiological parameter of a patient;
      detect an adaptive recognition event based on the operational data;
   based on the adaptive recognition event, determine an automation assistance parameter for the first surgical element, wherein the automation assistance parameter indicates a set of automated tasks that are performed by the first surgical element;
      transmit an indication of the automation assistance parameter to the first surgical element; and
      cause the first surgical element to perform the second set of automated tasks based on the automation assistance parameter.
17. The system of Example 16, wherein the adaptive recognition event comprises a determination that a physiological parameter of the patient satisfies a life-threatening threshold or a determined relationship between the first surgical element and the second surgical element.
18. The system of Example 16, wherein the processor is further configured to:
   send an automation strategy message to a user, wherein the automation strategy message comprises a request to perform an automated tasks associated with the automation assistance parameter; and
   receive a user input comprising an instruction to perform the automated task.
19. The system of Example 16, wherein the processor is further configured to:
   based on the adaptive recognition event, select a second automation assistance parameter, wherein the second automation assistance parameter indicates a second automated task.
20. The system of Example 19, wherein the processor is further configured to:
   transmit, to the second surgical element, an indication of the second automation assistance parameter; and
cause the second surgical element to perform the second automated task.

## Claims

1. A system to adaptively recognize an automation strategy during a procedure, the system comprising:
a processor configured to:
obtain operational data for a first surgical element and a second surgical element, wherein the operational data comprises an indication of a physiological parameter of a patient during the procedure, and a plurality of automated tasks associated with the procedure;
determine an automation assistance parameter for the first surgical element, wherein the automation assistance parameter is associated with a set of automated tasks from the plurality of automated tasks that are performed by the first surgical element during the procedure;
detect an adaptive recognition event, wherein the adaptive recognition event is at least one of:
a relationship between the first surgical element and the second surgical element; or
a determination that the physiological parameter of the patient satisfies a life-threatening threshold;
based on the adaptive recognition event, select a second automation assistance parameter for the first surgical element, wherein the second automation assistance parameter indicates a second set of automated tasks that are performed by the first surgical element during the procedure;
transmit an indication of the second automation assistance parameter to the first surgical element; and
cause the first surgical element to perform the second set of automated tasks based on the second automation assistance parameter.

2. A computer-readable medium comprising instructions which, when executed by a computer, cause the computer to:
obtain operational data for a first surgical element and a second surgical element, wherein the operational data comprises an indication of a physiological parameter of a patient during the procedure, and a plurality of automated tasks associated with the procedure;
determine an automation assistance parameter for the first surgical element, wherein the automation assistance parameter is associated with a set of automated tasks from the plurality of automated tasks that are performed by the first surgical element during the procedure;
detect an adaptive recognition event, wherein the adaptive recognition event is at least one of:
a relationship between the first surgical element and the second surgical element; or
a determination that the physiological parameter of the patient satisfies a life-threatening threshold;
based on the adaptive recognition event, select a second automation assistance parameter for the first surgical element, wherein the second automation assistance parameter indicates a second set of automated tasks that are performed by the first surgical element during the procedure;
transmit an indication of the second automation assistance parameter to the first surgical element; and
cause the first surgical element to perform the second set of automated tasks based on the second automation assistance parameter.

3. The system of claim 1 or computer-readable medium of claim 2, wherein:
the first surgical element is a ventilator;
the second surgical element is a heating device;
the physiological parameter of a patient is a CO2 percentage from the ventilator or a core body temperature from the heating device;
the procedure of the patient comprises a cardiac atrial fibrillation (AFIB) nerve ablation;
the adaptive recognition event comprises a determination that a CO2 to O2 or a core body temperature of the patient satisfies the life-threatening threshold; and
wherein the second automation assistance parameter comprises an instruction to decrease an O2 supplementation level of the ventilator or an instruction to increase a tidal volume of the ventilator.

4. The system or computer-readable medium of any preceding claim, wherein the physiological parameter of the patient satisfies a life-threatening threshold if the first surgical element or the second surgical element determines that: a heart rate exceeds an operational window of 40-120 beats per minute, an oxygen saturation of the patient decreases below 90%, or a core body temperature of the patient is less than 89 degrees Fahrenheit.

5. The system or computer-readable medium of any preceding claim , wherein the adaptive recognition event comprises the determination that the physiological parameter of the patient satisfies the life-threatening threshold, and wherein the selected second automation assistance parameter indicates that the second set of automated tasks comprises the plurality of automated tasks.

6. The system or computer-readable medium of any preceding claim , wherein the adaptive recognition event comprises the relationship between the first surgical element and the second surgical element, and wherein the selected second automation assistance parameter further comprises an instruction to perform the second set of automated tasks in response to a user input.

7. The system or computer-readable medium of claim 6, wherein the processor is further configured to, or the instructions further cause the computer to:
send an automation strategy message to a user, wherein the automation strategy message comprises a request to perform the second set of automated tasks; and
receive a user input comprising an instruction to perform at least one automated task of the second set of automated tasks.

8. The system or computer-readable medium of any preceding claim, wherein the processor is further configured to, or the instructions further cause the computer to:
receive position data associated with a movement of a user during the procedure and control data associated with the first surgical element and the second surgical element; and
determine the relationship between the first surgical element and the second surgical element based on the position data, the operational data, and the physiological parameter of the patient.

9. The system or computer-readable medium of any preceding claim, wherein the processor is further configured to, or the instructions further cause the computer to:
based on the adaptive recognition event, select an automation assistance parameter for the second surgical element, wherein the automation assistance parameter for the second surgical element indicates a set of automated tasks to be performed by the second surgical element during the procedure;
transmit, to the second surgical element, an indication of the automation assistance parameter for the second surgical element; and
cause the second surgical element to perform the set of automated tasks based on the automation assistance parameter for the second surgical element.

10. A system to adaptively recognize an automation strategy, the system comprising:
a processor configured to:
obtain operational data for a first surgical element and a second surgical element, wherein the operational data comprises an indication of a physiological parameter of a patient;
detect an adaptive recognition event based on the operational data;
based on the adaptive recognition event, determine an automation assistance parameter for the first surgical element, wherein the automation assistance parameter indicates a set of automated tasks that are performed by the first surgical element;
transmit an indication of the automation assistance parameter to the first surgical element; and
cause the first surgical element to perform the second set of automated tasks based on the automation assistance parameter.

11. A computer-readable medium comprising instructions which, when executed by a computer, cause the computer to:
obtain operational data for a first surgical element and a second surgical element, wherein the operational data comprises an indication of a physiological parameter of a patient;
detect an adaptive recognition event based on the operational data;
based on the adaptive recognition event, determine an automation assistance parameter for the first surgical element, wherein the automation assistance parameter indicates a set of automated tasks that are performed by the first surgical element;
transmit an indication of the automation assistance parameter to the first surgical element; and
cause the first surgical element to perform the second set of automated tasks based on the automation assistance parameter.

12. The system of claim 10 or computer-readable medium of claim 11, wherein the adaptive recognition event comprises a determination that a physiological parameter of the patient satisfies a life-threatening threshold or a determined relationship between the first surgical element and the second surgical element.

13. The system or computer-readable medium of any of claims 10 to 12, wherein the processor is further configured to, or the instructions further cause the computer to:
send an automation strategy message to a user, wherein the automation strategy message comprises a request to perform an automated tasks associated with the automation assistance parameter; and
receive a user input comprising an instruction to perform the automated task.

14. The system or computer-readable medium of any of claims 10 to 13, wherein the processor is further configured to, or the instructions further cause the computer to:
based on the adaptive recognition event, select a second automation assistance parameter, wherein the second automation assistance parameter indicates a second automated task.

15. The system or computer-readable medium of claim 14, wherein the processor is further configured to, or the instructions further cause the computer to:
transmit, to the second surgical element, an indication of the second automation assistance parameter; and
cause the second surgical element to perform the second automated task.
